# EUROPEAN PATENT SPECIFICATION

(11) **EP 3 501 506 B1**
(45) Date of publication and mention of the grant of the patent: **09.10.2019**
(21) Application number: 18154394.3
(22) Date of filing: 31.01.2018
(51) Int. Cl.: A61K 9/20, A61P 25/18, A61K 31/496

(54) **PHARMACEUTICAL TABLET COMPOSITION COMPRISING BREXPIPRAZOLE**
PHARMAZEUTISCHE TABLETTENZUSAMMENSETZUNG MIT BREXPIPRAZOL
COMPOSITION DE COMPRIMÉ PHARMACEUTIQUE COMPRENANT DU BREXPIPRAZOLE

(30) Priority: 19.12.2017 IN 201711045613
(43) Date of publication of application: 26.06.2019
(73) Proprietor: Alfred E. Tiefenbacher (GmbH & Co. KG), 22767 Hamburg (DE)
(72) Inventor: MUPKALKER, Ashwin Kumar, 502 319 Telangana (IN); KALAMATA, Venkatasimhadri Naidu, 502 319 Telangana (IN); RALLABANDI, Bala Ramesha Chary, 502 319 Telangana (IN); NADELLA, Prasad, 502 319 Telangana (IN); BANDLA, Srimannarayana, 502 319 Telangana (IN); STAVER, Ruslan, 22607 Hamburg (DE); SCHLEHAHN, Hendrik, 23843 Travenbrueck (DE)
(74) Representative: Hertin und Partner Rechts- und Patentanwälte PartG mbB

(56) References cited:
- EP-A1- 2 767 285
- WO-A1-2012/137971
- WO-A1-2016/003194

## Description

The invention relates to a pharmaceutical composition in the form of a tablet, comprising a) brexpiprazole as active ingredient and b) at least 5% by weight (wt%) pregelatinized starch (PGS), wherein said composition is prepared by a method comprising dry granulation of a blend of the components to produce granules, and compression of the granules to a tablet. The invention further relates to methods of preparing a pharmaceutical composition in the form of a tablet, comprising dry granulation of a blend of the components to produce granules and compression of the granules to a tablet. The invention further relates to the use of the composition in the treatment of schizophrenia, major depression, and other central nervous system disorders.

### BACKGROUND OF THE INVENTION

Brexpiprazole is an oral dopamine D2/D3 receptor and 5-HT1a partial agonist and 5HT2a and noradrenaline alphalB antagonist. Brexpiprazole has been described for use in the treatment of schizophrenia, depression and other central nervous system disorders, and for use in the prophylaxis and/or treatment of behavioral and psychological symptoms associated with neurodegenerative disease or impulsive symptoms associated with mental disease.

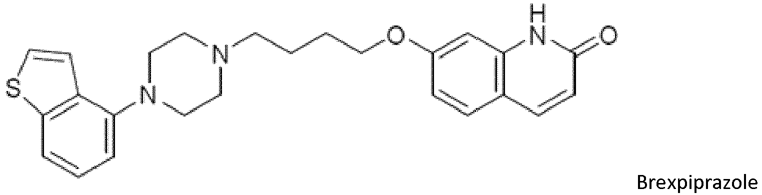

Brexpiprazole was developed by Otsuka and Lundbeck and has been approved by the FDA (Rexulti) for use as an adjunctive therapy to antidepressants for the treatment of major depressive disorder (MDD) and for the treatment of schizophrenia. The recommended starting dosage for Rexulti as an adjunctive treatment is 0.5 mg or 1 mg once daily. Dosgaes may be titrated to 1 mg once daily, then up to the target dosage of 2 mg once daily. The maximum recommended daily dosage is 3 mg.

WO 2006/112464 describes brexpiprazole, it analogues, and their use in the treatment of schizophrenia, major depression, and other central nervous system disorders. US 8,349,840 describes pharmaceutical compositions comprising brexpiprazole or it analogues. US 8,618,109 describes a method for treating schizophrenia by administering brexpiprazole or it analogues. WO 2014/065437 describes the use of brexpiprazole for the prophylaxis and/or treatment of behavioral and psychological symptoms associated with neurodegenerative disease or impulsive symptoms associated with mental disease. WO 2012/137971 discloses tablets comprising brexpiprazole, which are prepared either by direct compression or wet granulation.

WO 2013/054872 describes additional pharmaceutical formulations comprising brexpiprazole and methods for their manufacture. In particular, WO 2013/054872 describes producing uncoated or coated tablets of brexpiprazole using the following components: Brexpiprazole (0.25 mg), Lactose (48.15 mg), Corn starch (20.0 mg), Microcrystalline cellulose (10.0 mg), Low-substituted hydroxypropylcellulose (10.0 mg), Hydroxypropylcellulose (1.0 mg) and Magnesium stearate (0.6 mg). The compositions described therein are prepared by wet granulation techniques.

The prior art teaches that relatively complicated wet granulation methodologies are required in order to obtain suitable brexpiprazole formulations. In light of the formulations described in the art, a need exists for providing brexpiprazole formulations with acceptable content uniformity, stability and dissolution properties, without the need to employ the complicated multi-step wet granulation methods, as have been suggested previously.

### SUMMARY OF THE INVENTION

In light of the prior art the technical problem underlying the invention was the provision of improved or alternative means for pharmaceutical compositions comprising brexpiprazole that do not exhibit the disadvantages of the prior art.

This problem is solved by the features of the independent claims. Preferred embodiments of the present invention are provided by the dependent claims.

Therefore, the invention relates to a pharmaceutical composition in the form of a tablet, comprising a) brexpiprazole as active ingredient and b) at least 5% by weight (wt%) pregelatinized starch (PGS) wherein said composition is prepared by a method comprising dry granulation of a blend of the components to produce granules, and compression of the granules to a tablet.

The present invention is based on the finding that PGS functions in a surprisingly beneficial manner in combination with brexpiprazole in tablet formulations. As is outlined in more detail below, the use of PGS has enabled dry granulation formulation processes prior to tablet compression and additionally leads to beneficial content uniformity, stability and dissolution properties, without the need for wet granulation of the use of traditional disintegrants and/or binders, such as hydroxypropyl cellulose.

In some embodiments, the invention relates to a pharmaceutical composition in the form of a tablet, comprising a) brexpiprazole and b) 5% or more by weight (wt%) pregelatinized starch (PGS), such as 6 wt% or more, 7, 8, 9, 10, 11, 12, 13, 14, 15, 16, 17, 18, 19, 20, 25, 30, 35, 40, 45, 50, 55, 60 or 65 wt% or more.

In one embodiment, the pharmaceutical composition as described herein is free of hydroxypropyl cellulose (as a binder). In some embodiments, the presence of PGS enables the production of a tablet without hydroxypropyl cellulose. Hydroxypropyl cellulose is described as a key component in wet granulation methods and resulting formulations, as described in WO 2013/054872. In some embodiments, the pharmaceutical composition as described herein is free of low-substituted hydroxypropylcellulose. In preferred embodiments, the absence of hydroxypropylcellulose relates to the absence of the hydroxypropylcellulose from the tablet core, ie the hydroxypropylcellulose is not required as a binder. In some embodiments, hydroxypropyl methyl cellulose (HPMC, hypromellose) is employed as a coating agent.

In one embodiment of the invention, the composition is prepared by dry granulation of a blend of the components to produce granules, and compression of the granules to a tablet.

The preferred method of manufacture described herein, namely preparation by dry granulation and tablet compression, can lead to inherent structural differences compared to those methods described in the prior art. As such, the description of the tablet via its method of manufacture by dry granulation and compression enables a structural characterization and demarcation from other tablet formulations described in the art.

In some embodiments, the pharmaceutical composition as described herein is characterized in that the active ingredient has a particle size distribution (D90) of less than or equal to 20 µm. In some embodiments, the particle size distribution (D90) of the API may be 15 µm or less, 12 µm or less, or 10 µm or less. In some embodiments, the preferred particle size distribution (D90) is about 10 µm.

In some embodiments, the particle size distribution (D90) of the API brexpiprazole may however be larger, for example in some embodiments the particle size distribution (D90) is between 50 and 500 µm, preferably between 100 and 400 µm, more preferably between 200 and 300 µm. In some embodiments, the particle size distribution (D90) is about 250 µm.

As described in more detail below, the formulation according to BRE/F3 uses a more coarse API preparation, with a particle size distribution (D90) of about 250 µm. Similarly, the formulation BRE/C1, which reflects a combination of very similar components to the commercial formulation, also uses the coarser API preparation. Surprisingly, the formulation according to BRE/F3 shows improved dissolution values in comparison to BRE/C1. Therefore, one additional advantage of the present PGS-comprising compositions is that coarse API may be employed with acceptable dissolution properties, thereby removing the need for finer and therefore more complicated and cost-intensive API preparations. Although employing a coarser grade of API is not necessarily preferred, the use of PGS enables a skilled person to consider a greater variety of API preparations and particle size distribution values when producing brexpiprazole tablets.

In some embodiments, the pharmaceutical composition further comprises a water-soluble filler, a water-insoluble filler and a lubricant.

In one embodiment, the water-soluble filler is a sugar, preferably lactose. Other sugar water-soluble fillers relate to mannitol, sorbitol, xylitol, and the like, and mixtures thereof; preferably lactose.

In one embodiment, the water-insoluble filler is microcrystalline cellulose. Other water-insoluble fillers include but are not limited to starch, powdered cellulose, calcium phosphate and the like, or combinations thereof.

In one embodiment, the lubricant is magnesium stearate.

In one embodiment, the tablet is coated, preferably wherein the coating is a film coating comprising hydroxypropyl methyl cellulose (HPMC, hypromellose), preferably present in an amount of 0.1-5 wt% based on the total weight of all components of the tablet. Alternatively, poly(vinyl alcohol) (PVA) may be employed in the coating.

In further preferred embodiments, the pharmaceutical composition as described herein comprises:
a. brexpiprazole, present in an amount of 0.01-25 wt%, preferably 1-10 wt%;
b. pregelatinized starch, present in an amount of 5-70 wt%, preferably 15-60 wt%;
c. water-soluble filler, preferably a sugar, more preferably lactose, present in an amount of 10-99 wt%, preferably 15-50 wt%;
d. water-insoluble filler, preferably microcrystalline cellulose, present in an amount of 5-70 wt%, preferably 5-50 wt%; and
e. lubricant, preferably magnesium stearate, present in an amount of 0.01-5 wt%, preferably 0.1-2 wt%, based on the total weight of all components of the tablet.

Additional preferred embodiments of the composition relate preferably to BRE/F1, BRE/F2, BRE/F3, BRE/F4 or alternative formulations based closely on these embodiments, as demonstrated below, in particular compositions with quantities of components falling within the ranges indicated by the "% range" parameters disclosed below.

The below embodiments may also be considered to encompass further embodiments of the invention in which the indicated amounts of the components are employed, but alternative water-soluble fillers, water insoluble fillers and/or lubricants, other than the specific components disclosed below, are used.

| **BRE/F1** | | | | |
|---|---|---|---|---|
| **S. No.** | **Ingredients** | **mg/tab** | **wt%** | **Preferred wt% range** |
| **Stage-A (Sifting & Blending & Slugging)** | | | | |
| 1 | Brexpiprazole, preferably *(D₍₉₀₎ <10 µm)* | 4 | 4.3% | 1-10% |
| 2 | Lactose, preferably anhydrous (Supertab 21 AN) | 30.3 | 32.6% | 20-50% |
| 3 | Cellulose, Microcrystalline (preferably Comprecel® M 102 D+) | 30.3 | 32.6% | 20-50% |
| 4 | Pregelatinized Starch (preferably PC-10) | 25 | 26.9% | 15-40% |

| **Stage-B (Lubrication)** | | | | |
|---|---|---|---|---|
| 1 | Magnesium Stearate | 0.4 | 0.4% | 0.05-2% |
| **Core tablet weight** | | **90** | | |

| **Stage-C (Coating)** | | | | |
|---|---|---|---|---|
| 1 | Coating agent, preferably Opadry® 03A580013 White | 3 | 3.2% | 1-5% |
| 2 | Water, purified | q.s. | | |
| **Coated tablet weight** | | **93** | 100.0% | 100.0% |

| **BRE/F2** | | | | |
|---|---|---|---|---|
| **S. No.** | **Ingredients** | **mg/tab** | **wt%** | **Preferred wt% range** |
| **Stage-A (Sifting & Blending & Slugging)** | | | | |
| 1 | Brexpiprazole, preferably *(D₍₉₀₎ <10 µm)* | 4 | 4.3% | 1-10% |
| 2 | Lactose, preferably anhydrous (Supertab 21 AN) | 25.6 | 27.5% | 20-50% |
| 3 | Cellulose, Microcrystalline (preferably Comprecel® M 102 D+) | 10 | 10.8% | 5-30% |
| 4 | Pregelatinized Starch (preferably PC-10) | 50 | 53.8% | 30-70% |

| **Stage-B (Lubrication)** | | | | |
|---|---|---|---|---|
| 1 | Magnesium Stearate | 0.4 | 0.4% | 0.05-2% |
| **Core tablet weight** | | **90** | | |

| **Stage-C (Coating)** | | | | |
|---|---|---|---|---|
| 1 | Coating agent, preferably Opadry® 03A580013 White | 3 | 3.2% | 1-5% |
| 2 | Water, purified | q.s. | | |
| **Coated tablet weight** | | **93** | 100.0% | 100.0% |

| **BRE/F3** | | | | |
|---|---|---|---|---|
| **S. No.** | **Ingredients** | **mg/tab** | **wt%** | **Preferred wt% range** |
| **Stage-A (Sifting & Blending & Slugging)** | | | | |
| 1 | Brexpiprazole, preferably (D_{(0.9)}≈250µm) | 4 | 4.3% | 1-10% |
| 2 | Lactose, preferably anhydrous (Supertab 21 AN) | 25.4 | 27.3% | 15-50% |
| 3 | Cellulose, Microcrystalline (preferably Comprecel® M 102 D+) | 10 | 10.8% | 5-30% |
| 4 | Pregelatinized Starch (preferably PC-10) | 50 | 53.8% | 30-70% |

| **Stage-B (Lubrication)** | | | | |
|---|---|---|---|---|
| 1 | Magnesium Stearate | 0.6 | 0.6% | 0.05-2% |
| **Core tablet weight** | | **90** | | |

| **Stage-C (Coating)** | | | | |
|---|---|---|---|---|
| 1 | Coating agent, preferably Opadry® 03A580013 White | 3 | 3.2% | 1-5% |
| 2 | Water, purified | q.s. | | |
| **Coated tablet weight** | | **93** | 100.0% | 100.0% |

| | | | | |
|---|---|---|---|---|
| **Strength** | | **0.25 mg** | | |

| **BRE/F4** | | | | |
|---|---|---|---|---|
| **S. No.** | **Ingredients** | **mg/tab** | **wt%** | **Preferred wt% range** |
| **Stage-A (Sifting & Blending & Slugging)** | | | | |
| 1 | Brexpiprazole, preferably *(D₍₉₀₎ <10 µm)* | 0.25 | 0.3% | 0.05-2% |
| 2 | Lactose, preferably anhydrous (Supertab 21 AN) | 32.175 | 34.6% | 15-50% |
| 3 | Cellulose, Microcrystalline (preferably Comprecel® M 102 D+) | 32.175 | 34.6% | 15-50% |
| 4 | Pregelatinized Starch (preferably PC-10) | 25 | 26.9% | 15-45% |

| **Stage-B (Lubrication)** | | | | |
|---|---|---|---|---|
| 1 | Magnesium Stearate | 0.4 | 0.4% | 0.05-2% |
| **Core tablet weight** | | **90** | | |

| **Stage-C (Coating)** | | | | |
|---|---|---|---|---|
| 1 | Coating agent, preferably Opadry® 03A565009 Brown | 3 | 3.2% | 1-5% |
| 2 | Water, purified | q.s. | | |
| **Coated tablet weight** | | **93** | 100.0% | 100% |

In some embodiments, the composition may be defined by functional properties. In one embodiment, the tablet shows at least 75% dissolution of the active ingredient after 30 minutes using method 2 of dissolution test of the US Pharmacopeia (USP), with a paddle speed of 50 revolutions per minute in 900mL of 0.05 M Acetate buffer at pH 4.3.

In a further aspect, the invention relates to the pharmaceutical composition as described herein for use as a medicament in the treatment of schizophrenia, major depression, and other central nervous system disorders. The invention therefore relates to a method of treating schizophrenia, major depression, and other central nervous system disorders, comprising administering to a subject in need thereof a therapeutically effective amount of brexpiprazole in form of the composition of the present invention.

In a further aspect, the invention relates to a method of preparing a pharmaceutical composition in the form of a tablet, comprising dry granulation of a blend of the components to produce granules and compression of the granules to a tablet.

In a preferred embodiment, the method of preparing a pharmaceutical composition in the form of a tablet comprises:
a. blending of the components of the tablet core to produce a blend;
b. optionally compacting said blend into flakes;
c. milling the blend (or optionally the flakes) to produce granules;
d. lubricating the granules; and
e. compression of the granules to a tablet.

In one embodiment, the method of preparing a pharmaceutical composition in the form of a tablet comprises additionally coating said tablet with a film coating, preferably comprising hydroxypropyl methyl cellulose (HPMC, hypromellose).

In one embodiment of the invention, the method for preparing the pharmaceutical composition is characterized in that the method comprises:
- Dispensing the blend components of the core, preferably brexpiprazole, pregelatinized starch, water-soluble filler and water-insoluble filler;
- Sifting the brexpiprazole and the filler(s) with PGS as a blend through a suitable mesh;
- Optionally sifting the lubricant separately through a suitable mesh (this step may also be combined with the step above, enabling a single sifting step);
- Loading the materials above (preferably with the exception of the lubricant) in a blender and mixing for sufficient time to mix the components (pre-lubrication blending);
- Dry granulation of the blend, preferably using roller compaction;
- Optionally milling the granules in order to achieve the desired granule size;
- Optionally adding the lubricant (if not already incorporated, or adding the remaining portion of the lubricant) and continue the blending for sufficient time to mix the granules with the lubricant (lubrication blending);
- Compressing the granules into tablets using the (lubricated) granules of the previous step.

In some embodiments, the method comprises:
a. Sifting all core materials through a mesh, such as #30,
b. Blending materials of stage a. in a blender,
c. Compacting the blend into flakes,
d. Milling the flakes to produce the granules,
e. Lubricating the milled granules with magnesium stearate,
f. Compressing the lubricated blend into tablets, and
g. Coating the tablets using a coating agent, preferably a dispersion of Opadry® 12%, preferably in water.

The present invention therefore enables more straightforward and efficient methods of manufacture of a pharmaceutical tablet formulation for brexpiprazole, by incorporating dry granulation methods using PGS as an excipient, and subsequent compression into tablets. The disadvantages of wet granulation (longer processing/drying time/ use of additional solvents, etc) are thereby avoided, whilst the beneficial properties of the tablet are maintained.

### DETAILED DESCRIPTION OF THE INVENTION

Brexpiprazole is an oral dopamine D2/D3 receptor and 5-HT1a partial agonist and 5HT2a and noradrenaline alphalB antagonist and is known as 7-{4-[4-(1-benzothiophen-4-yl)piperazin-1-yl]butoxy}quinolin-2(1H)-one, or 7-[4-(4-benzo[b]thiophen-4-yl-piperazin-1-yl)butoxy]-1H-quinolin-2-one (CAS No.: 913611-97-9).

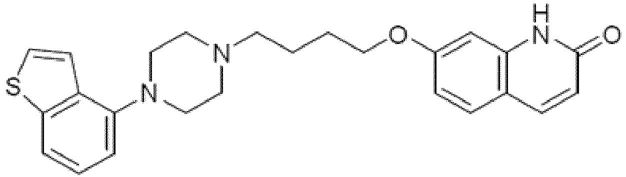

The term "brexpiprazole" as used herein according to the present invention includes brexpiprazole in the form of free base, a pharmaceutically acceptable salt thereof, amorphous brexpiprazole, crystalline brexpiprazole, preferably selected from the polymorph forms described herein, any isomer, derivative, hydrate, solvate or prodrug or a combination thereof.

Brexpiprazole may also be prepared as a pharmaceutical salt. Examples of pharmaceutical acceptable salts of brexpiprazole which can be contained as an active ingredient in a solid oral dosage form include acid addition salt formed with mineral acids such as hydrochloric acid, hydrobromic acid, hydroiodic acid, sulfuric acid, nitric acid, phosphoric acid and the like; acid addition salts formed with organic acids such as acetic acid, propionic acid, butyric acid, oxalic acid, citric acid, succinic acid, tartaric acid, fumaric acid, malic acid, lactic acid, adipic acid, benzoic acid, salicylic acid, methanesulfonic acid, benzenesulfonic acid, p-toluenesulfonic acid, glutamic acid, aspartic acid and the like. Examples of solvate include solvates with water, ethyl alcohol or the like.

In a preferred embodiment, the present invention employs a stable polymorphic form of brexpiprazole as used in commercially available Rexulti formulations. A skilled person is therefore capable of manufacturing and/or selecting an appropriate form of brexpiprazole for the composition of the present invention. For example, the stable polymorphic form of Brexpiprazole API is prepared according to the Comparative Example 1 of WO 2013162046 leading to neat API form with a XRD diffractogram essentially the same as disclosed in Fig. 12 of WO 2013162046 and having the characteristic peaks at about 14.4°, 19.1°, 20.2°, 21.3°, and 23.2° 20.

The term "active ingredient" or "API" herein refers to a pharmaceutically active molecule (e.g. brexpiprazole) as well as its pharmaceutically acceptable and therapeutically active salts, esters, amides, prodrugs, metabolites, enantiomers, polymorphs, analogs, etc. that induce a desired pharmacological or physiological effect. Terms like "active", "active agent", "active substance" may be used synonymously for "active ingredient".

The term "particle size distribution" as used herein refers to the statistical distribution of the volume share related of all particle sizes. Preferred embodiments of the invention use an active ingredient with a particle size distribution (D90) of less than or equal to 20 µm. Other embodiments employ API with a larger particle size, for example about 250 µm. Within this application, the D90 values are determined by the light scattering method, using a Mastersizer 2000 apparatus made by Malvern Instruments. A wet measurement on a dispersion of the particles in a dispersing agent at 25° C is preferred. The D90 value of the integral volume distribution is defined in the context of this invention as the particle diameter, at which 90 percent by volume of the particles have a smaller diameter than the diameter, which corresponds to the D90 value. The particle size distribution according to the invention can be monomodal or bimodal. In the preferred embodiment of the invention the particle size distribution of the active agent is monomodal. The term "monomodal" as used herein refers to only one peak observed in the histogram and/or a graph of the frequency distribution.

Tablets may be prepared, in some embodiments, with 0.25 mg, 0.5 mg, 1 mg, 2 mg, 3 mg, or 4 mg API per tablet, preferably in film-coated tablets.

The excipients are described herein in some embodiments according to "wt%", or "percentage by weight". The %wt values recite herein preferably relate to the percentage of material by weight present in the final tablet, or in the core tablet prior to coating. Preferably, the percentage indications relate to the wt% of the final tablet, including the mass of the tablet coating.

The term "excipient" means a pharmacologically inactive component such as a diluent, disintegrant, carrier, and the like, of a pharmaceutical product. The excipients that are useful in preparing a pharmaceutical composition are generally safe, non-toxic and are acceptable for veterinary as well as human pharmaceutical use. Reference to an excipient includes both one excipient and more than one excipient.

A disintegrant is typically an excipient used in the preparation of solid pharmaceutical formulations which causes them to disintegrate and release their medicinal substances on contact with moisture. However, the disintegrant may also be considered as a filler and/or binder, depending on the properties of the particular excipient employed as a disintegrant. A binder is typically a substance used to create a desired consistency in a formulation, whereby a filler is typically considered as a bulking agent to increase the mass of the formulation. Accordingly, the disintegrant exhibits properties of disintegration upon contact with aqueous environments.

Pregelatinized starch (PGS) is generally considered as a disintegrant, but can also be considered as a water-insoluble filler. PGS may also have binder-like properties. PGS is therefore a multi-functional excipient. PGS is a starch that has been chemically and/or mechanically processed to rupture all or part of the starch granules. This typically renders the starch flowable and directly compressible (Handbook of Pharmaceutical Excipients (Ed: Rowe)). Partially pregelatinized starch is commercially available. In some embodiments, pregelatinized starch contains 5% of free amylose, 15% of free amylopectin, and 80% unmodified starch. Pregelatinized starch is typically obtained from maize (corn), potato, or rice starch. Pregelatinized starch may be employed in granule or tablet formulations and shows multiple functions as a binder, diluent and/or disintegrant. PGS may also be used as a tablet binder in dry-compression or direct compression processes.

According to the invention, a diluent (or filler) may be used as a bulking agent. Preferred fillers are water soluble fillers, such as lactose. Various additional or alternative water-soluble fillers or diluents include but are not limited to mannitol, sorbitol, xylitol, and the like, and mixtures thereof; most preferably lactose.

According to the invention, a water-insoluble filler may be used. Various useful water-insoluble fillers include but are not limited to microcrystalline cellulose, starch, powdered cellulose, (MCC), calcium phosphate and the like, or combinations thereof.

According to the present invention, one or more lubricants can be used. Useful lubricants include but are not limited to stearates, such as magnesium stearate, or sodium stearyl fumarate.

A "tablet" as used herein is considered a solid unit dosage form of a medicament comprising one or more excipients.

The most commonly used pharmaceutical solid dosage forms today include granules, pellets, tablets and capsules. Tablets are solid pharmaceutical dosage forms containing drug substances with one or more excipients prepared by either compression or molding methods. The basic art of tableting by three well known methods includes direct compression, wet granulation and dry granulation.

According to the present invention dry granulation is employed. Dry granulation may typically be employed if the materials have sufficient inherent binding or cohesive properties to form granules. Dry granulation refers typically to the process of granulating without the use of liquids. Two dry granulation methods are primarily used in the pharmaceutical industry, namely slugging and roller compaction. In a roller compactor material particles are consolidated and densified by passing the material between two high pressure rollers. The densified material from a roller compactor is then reduced to a uniform granule size by milling. Roll compaction/dry granulation (RCDG) is a method of choice for processing of physically or chemically moisture sensitive drugs, as no liquid binder is required in the granulation, and is a preferred method of the present invention.

In the dry granulation method of tablet production, dry ingredients are thoroughly mixed to a blend, the blend is granulated, for example by roller compaction and granulation and optionally milling, and the granules are then compressed into tablets. This eliminates the drying steps associated with the wet granulation method. It also reduces the higher costs involved in wet granulation including increased equipment, labor, time, process validation and energy expenditure. As a result, dry granulation is both efficient and economical, well suited to the production of high quality tablets, which exhibit hardness, low friability and excellent dissolution rates. As an added benefit, dry granulation can improve the physical and chemical stability of tablets as compared to wet granulation.

Also disclosed herein are methods of direct compression of the blend of core materials. In the direct compression method of tablet production, dry ingredients are thoroughly mixed to a powder blend and then compressed into tablets. This eliminates the drying steps associated with the wet granulation method. It also reduces the higher costs involved in wet granulation including increased equipment, labor, time, process validation and energy expenditure. As a result, direct compression is both efficient and economical, well suited to the production of high quality tablets, which exhibit hardness, low friability and excellent dissolution rates. As an added benefit, direct compression can improve the physical and chemical stability of tablets as compared to wet granulation.

Methods for compression of tablets are known to a skilled person and may be elected appropriately without undue effort. For example, after mixing of ingredients (in case of direct compression), the powder blend may be directly compressed to obtain a tablet. Methods for compression of granules into tablets are also known to a skilled person, wherein granules are subsequently compressed into tablets. The compression is preferably carried out by either by single punch machine (stamping press) or by multi station machine (rotary press). The tablet press is a high-speed mechanical device. It 'squeezes' the ingredients into the required tablet shape with extreme precision. It can make the tablet in many shapes, although they are usually round or oval. Also, it can press the name of the manufacturer or the product into the top of the tablet.

The term "effective amount" or "therapeutically effective amount" used interchangeably, is defined to mean the amount or quantity of the active drug (e.g. brexpiprazole), which is sufficient to elicit an appreciable biological response when administered to the patient. It will be appreciated that the precise therapeutic dose will depend on the age and condition of the patient, nature of the condition to be treated and will be at the ultimate discretion of the attendant physician.

Accordingly, the compositions of the present invention are effective for the treatment or prevention of central nervous system disorders including the group consisting of schizophrenia; refractory, intractable or chronic schizophrenia; emotional disturbance; psychotic disorder; mood disorder; bipolar disorder (for example, bipolar I type disorder and bipolar II type disorder) ; depression; endogenous depression; major depression; melancholy and refractory depression; dysthymic disorder; cyclothymic disorder; anxiety disorder (for example, panic attack, panic disorder, agoraphobia, social phobia, obsessive- compulsive disorder, post-traumatic stress disorder, generalized anxiety disorder, acute stress disorder, etc.); somatoform disorder (for example, hysteria, somatization disorder, conversion disorder, pain disorder, hypochondriasis, etc.); factitious disorder; dissociative disorder; sexual disorder (for example, sexual dysfunction, sexual desire disorder, sexual arousal disorder, erectile dysfunction, etc.); eating disorder (for example, anorexia nervosa, bulimia nervosa, etc.); sleep disorder; adjustment disorder; substance-related disorder (for example, alcohol abuse, alcohol intoxication, drug addiction, stimulant intoxication, narcotism, etc.); anhedonia (for example, iatrogenic anhedonia, anhedonia of a psychic or mental cause, anhedonia associated with depression, anhedonia associated with schizophrenia, etc.); delirium; cognitive impairment; cognitive impairment associated with Alzheimer's disease, Parkinson's disease, and other neurodegenerative diseases; cognitive impairment caused by Alzheimer's disease, Parkinson's disease and associated neurodegenerative diseases; cognitive impairment of schizophrenia; cognitive impairment caused by refractory, intractable or chronic schizophrenia; vomiting; motion sickness; obesity; migraine; pain (ache) ; mental retardation; autism disorder (autism); Tourette's disorder; tic disorder; attention-deficit/hyperactivity disorder; conduct disorder; and Down's syndrome. Furthermore, the compounds of the present, invention have little or no side effects and they are excellent in safety and tolerability. Mental disorders may also be treated, such as mood disorders including depression and major depressive disorder, bipolar disorder, anxiety disorder, schizophrenia and the like.

### EXAMPLES

The invention is demonstrated by way of the examples disclosed herein. The examples provide technical support for and a more detailed description of potentially preferred embodiments of the invention.

### Part A

### Comparative examples

### Comparative example BRE/C1:

| **Batch No.** | | **BRE/C1** |
|---|---|---|
| **S. No.** | **Ingredients** | **mg/tab** |
| **Stage-A (Dry mix)** | | |
| 1 | Brexpiprazole (D_{(0.9)}≈250µm) | 4.0 |
| 2 | Lactose | 44.4 |
| 3 | Corn Starch | 20.0 |
| 4 | Microcrystalline Cellulose | 10.0 |
| 5 | Low-Substituted Hydroxypropylcellulose (LHPC LH-11) | 10.0 |

| **Stage-B (Binder solution)** | | |
|---|---|---|
| 1 | Hydroxypropylcellulose | 1.0 |
| 2 | Water, Purified | q.s. |

| **Stage-C (Lubrication)** | | |
|---|---|---|
| 1 | Magnesium Stearate | 0.6 |
| **Core tablet weight** | | **90.0** |

| **Stage-D (Coating)** | | |
|---|---|---|
| 1 | Opadry® 03A580013 White | 3.0 |
| 2 | Water, purified | q.s. |
| **Coated tablet weight** | | **93.0** |

| | | |
|---|---|---|
| ** Remarks: Capping was observed.* | | |

Manufacturing procedure is wet granulation process, as disclosed in the innovator's patent application EP 2 767 285 A1.

### Comparative Example BRE/C2:

| | **Batch No.** | **BRE/C2** |
|---|---|---|
| **S. No.** | **Ingredients** | **mg/tab** |
| **Stage-A (Sifting & Blending & Slugging)** | | |
| 1 | Brexpiprazole *(D₍₉₀₎ <10 µm)* | 4.00 |
| 2 | Lactose anhydrous (Supertab 21 AN) | 25.70 |
| 3 | Cellulose, Microcrystalline (Comprecel® M 102 D+) | 59.90 |

| **Stage-B (Lubrication)** | | |
|---|---|---|
| 1 | Magnesium Stearate | 0.400 |
| **Core tablet weight** | | **90.00** |

| **Stage-C (Coating)** | | |
|---|---|---|
| 1 | Opadry® 03A580013 White | 3.00 |
| 2 | Water, purified | q.s. |
| **Coated tablet weight** | | **93.00** |

Manufacturing procedure is dry granulation process, same as for the inventive examples below.

### Example BRE/C3:

| **Batch No.** | | **BRE/C3** |
|---|---|---|
| **S. No.** | **Ingredients** | **mg/tab** |
| **Stage-A (Sifting & Blending & Slugging)** | | |
| 1 | Brexpiprazole *(D₍₉₀₎ <10 µm)* | 4.00 |
| 2 | Lactose anhydrous (Supertab 21 AN) | 44.40 |
| 3 | Cellulose, Microcrystalline (Comprecel® M 102 D+) | 36.50 |
| 4 | Pregelatinized Starch (PC-10) | 4.50 |

| **Stage-B (Lubrication)** | | |
|---|---|---|
| 1 | Magnesium Stearate | 0.600 |
| **Core tablet weight** | | **90.00** |

| **Stage-C (Coating)** | | |
|---|---|---|
| 1 | Opadry® 03A580013 White | 3.00 |
| 2 | Water, purified | q.s. |
| **Coated tablet weight** | | **93.00** |

### Part B

### General procedure for the preparation of inventive examples below employing a dry granulation process:

1. All the materials were sifted through mesh #30.
2. Materials of stage-A were blended in a blender and the blend was compacted into flakes.
3. The compacts/flakes were milled to produce the granules and the milled granules were lubricated with magnesium stearate.
4. The lubricated blend was compressed into tablets and the tablets were coated using 12% dispersion of Opadry® in water.

The following compositions were obtained using the above process:

### Inventive examples:

### Example BRE/F1:

| **Batch No.** | | **BRE/F1** |
|---|---|---|
| **S. No.** | **Ingredients** | **mg/tab** |
| **Stage-A (Sifting & Blending & Slugging)** | | |
| 1 | Brexpiprazole *(D₍₉₀₎ <10 µm)* | 4.00 |
| 2 | Lactose anhydrous (Supertab 21 AN) | 30.30 |
| 3 | Cellulose, Microcrystalline (Comprecel® M 102 D+) | 30.30 |
| 4 | Pregelatinized Starch (PC-10) | 25.00 |

| **Stage-B (Lubrication)** | | |
|---|---|---|
| 1 | Magnesium Stearate | 0.400 |
| **Core tablet weight** | | **90.00** |

| **Stage-C (Coating)** | | |
|---|---|---|
| 1 | Opadry® 03A580013 White | 3.00 |
| 2 | Water, purified | q.s. |
| **Coated tablet weight** | | **93.00** |

### Example BRE/F2:

| **Batch No.** | | **BRE/F2** |
|---|---|---|
| **S. No.** | **Ingredients** | **mg/tab** |
| **Stage-A (Sifting & Blending & Slugging)** | | |
| 1 | Brexpiprazole *(D₍₉₀₎ <10 µm)* | 4.00 |
| 2 | Lactose anhydrous (Supertab 21 AN) | 25.60 |
| 3 | Cellulose, Microcrystalline (Comprecel® M 102 D+) | 10.00 |
| 4 | Pregelatinized Starch (PC-10) | 50.00 |

| **Stage-B (Lubrication)** | | |
|---|---|---|
| 1 | Magnesium Stearate | 0.400 |
| **Core tablet weight** | | **90.00** |

| **Stage-C (Coating)** | | |
|---|---|---|
| 1 | Opadry® 03A580013 White | 3.00 |
| 2 | Water, purified | q.s. |
| **Coated tablet weight** | | **93.00** |

### Example BRE/F3:

| | **Batch No.** | **BRE/F3** |
|---|---|---|
| **S. No.** | **Ingredients** | **mg/tab** |
| **Stage-A (Sifting & Blending & Slugging)** | | |
| 1 | Brexpiprazole **(D_{(0.9)}≈250µm)** | 4.00 |
| 2 | Lactose anhydrous (Supertab 21 AN) | 25.40 |
| 3 | Cellulose, Microcrystalline (Comprecel® M 102 D+) | 10.00 |
| 4 | Pregelatinized Starch (PC-10) | 50.00 |

| **Stage-B (Lubrication)** | | |
|---|---|---|
| 1 | Magnesium Stearate | 0.600 |
| **Core tablet weight** | | **90.00** |

| **Stage-C (Coating)** | | |
|---|---|---|
| 1 | Opadry® 03A580013 White | 3.00 |
| 2 | Water, purified | q.s. |
| **Coated tablet weight** | | **93.00** |

### Part C

### Dissolution Study:

For the tablet formulations of inventive examples and comparative examples, the dissolution test was carried out according to the following dissolution test methods and conditions.

### Dissolution conditions and methods:

The test was carried out using tablets at a paddle speed of 50 revolutions per minute (RPM) according to method 2 (Paddle) of dissolution test of the USP, using 900 mL of 0.05 M acetate buffer at pH 4.3. The temperature of the medium is maintained at 37°C ± 0.5°C using a water bath. Sample solutions were obtained at 10, 15, 20, 30, and 45 minutes after starting the test, and filtered through a 0.45 µm PVDF Millipore syringe filter.

**Table: Comparative dissolution profiles of Brexpiprazole tablets 4 mg with Rexulti™ in 0.05 M Acetate buffer pH 4.3, Paddle-50 RPM, 900mL**

| **Time (min)** | **Mean cumulative % labelled amount dissolved** | | | | | | |
|---|---|---|---|---|---|---|---|
| | **Rexulti™ 4 mg (BPS00115B)** | **(D_{(0.9)}≈10µm)** | | | | **(D_{(0.9)}≈250µm)** | |
| | | **BRE/C2** | **BRE/C3** | **BRE/F1** | **BRE/F2** | **BRE/F3** | **BRE/C1** |
| 10 | 71 | 49 | 52 | 78 | 83 | 54 | 26 |
| 15 | 79 | 58 | 60 | 82 | 85 | 64 | 35 |
| 20 | 85 | 66 | 65 | 85 | 88 | 68 | 41 |
| 30 | 91 | 72 | 71 | 88 | 89 | 75 | 53 |
| 45 | 97 | 76 | 77 | 89 | 90 | 82 | 64 |

*The specification for dissolution in release medium (0.05 M Acetate buffer pH 4.3, Paddle-50 RPM, 900mL) is NLT 75% (Q) in 30 min.*

The experimental data of table above shows that 75 wt.% or more of Brexpiprazole of the inventive pharmaceutical tablet compositions is dissolved within 30 minutes in accordance with the regulatory specification. Furthermore, the dissolution of the inventive formulations of the present invention is comparable to the commercially available reference product (Rexulti™ 4 mg film-coated tablets).

The dissolution for the comparative examples BRE/C2, BRE/C3, and BRE/C1 is slower, and 75% drug release is not achieved at 30 min time point.

### Part D

### Assay and Content Uniformity of Tablets:

The suitability of the dry granulation process with regard to content uniformity in the lowest strength is evaluated. The content uniformity of tablets of 0.25 mg strength produced according to the invention was tested. The formulation according to unit formula below was employed. No difficulties like capping or sticking were observed.

### Example BRE/F4:

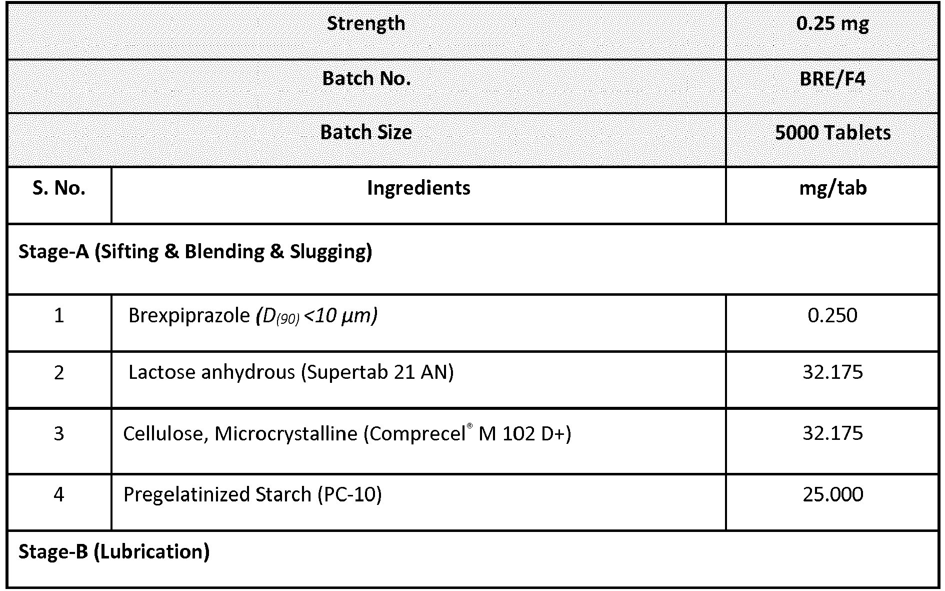

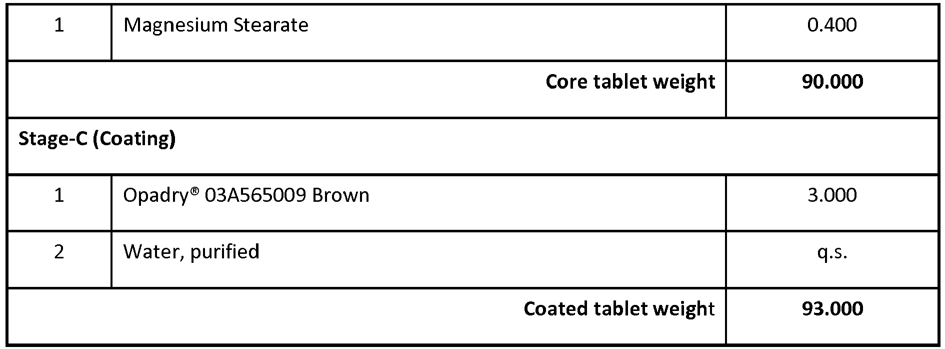

To ensure the consistency of dosage units, each unit in a batch should have an active substance content within a narrow range around the label claim. Dosage units are defined as dosage forms containing a single dose or a part of a dose of an active substance in each dosage unit. The term "Uniformity of dosage unit" is defined as the degree of uniformity in the amount of the active substance among dosage units. Therefore, the requirements of this regulation can be applied to any active substance being comprised in dosage units containing one or more active substances. The uniformity of dosage forms (by content uniformity) was assessed according to USP. The assay (%) of 10 individual tablets was detected by HPLC analysis.

There are no issues with content uniformity with respect to content uniformity in lower strength (0.25 mg) as can be seen in table below.

**Table: Results from assessment of content uniformity and assay of tablets**

| **Content Uniformity of dosage units** | **BRE/F4** |
|---|---|
| Tablet-1 | 97.5 |
| Tablet-2 | 101.3 |
| Tablet-3 | 97.0 |
| Tablet-4 | 97.1 |
| Tablet-5 | 102.0 |
| Tablet-6 | 100.2 |
| Tablet-7 | 99.1 |
| Tablet-8 | 98.4 |
| Tablet-9 | 98.9 |
| Tablet-10 | 100.4 |

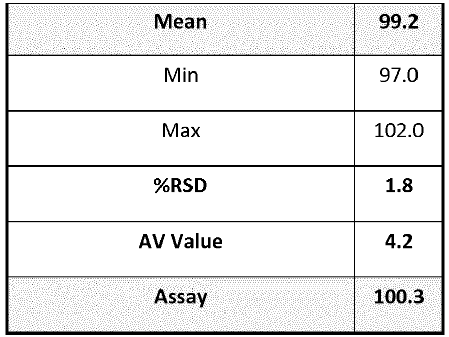

As can be observed from the data presented above, the content uniformity is found to be within the specification limits according to USP. The accepted maximum allowed acceptance value (AV Value) L1 = 15.0 was clearly satisfied by the batch BRE/F4, showing the AV value of 4.2, as mentioned in the above table. Furthermore, both the assay of tablets and the average amount of API contained per tablet (as described above under "Mean") demonstrate values of 100.3% and 99.2%, correspondingly, clearly satisfying the standard requirements of 95-105%.

### Part E

### ASAP Stability Study:

### Chemical Stability/Impurity profiles:

The formulation according to example BRE/F1 was tested under accelerated conditions (open exposure) for a defined time to test the presence of brexpiprazole degradation products.

Degradation products were tested prior to storage and after storage under conditions as shown in tables for the corresponding examples. Degradation products were detected by HPLC analysis.

The results for the formulation BRE/F1 are presented in the table below.

**Table. ASAP Stability data of Coated Tablets, Open Exposure: BRE/F1**

| **Condition** | **Days** | **% Known impurity** | | | | | | | **% Max. Unknown impurity** | **% Total impurities** |
|---|---|---|---|---|---|---|---|---|---|---|
| | | **Imp-1** | **Imp-2** | **Imp-3** | **Imp-4** | **Imp-5** | **Imp-6** | **Imp-7** | | |
| Initial | - | ND | ND | ND | ND | ND | ND | ND | 0.10 | 0.14 |
| 50°C/75%RH | 14 Days | ND | ND | ND | ND | ND | ND | ND | 0.10 | 0.20 |
| 60°C/40%RH | 14 Days | ND | ND | ND | ND | ND | ND | ND | 0.10 | 0.23 |
| 70°C/5%RH | 14 Days | 0.07 | ND | ND | ND | ND | ND | ND | 0.11 | 0.38 |
| 60°C/75%RH | 14 Days | ND | ND | ND | ND | ND | ND | ND | 0.10 | 0.19 |

| | | | | | | | | | | |
|---|---|---|---|---|---|---|---|---|---|---|
| *ND* = *not detected* | | | | | | | | | | |

## Claims

1. A pharmaceutical composition in the form of a tablet, comprising a) brexpiprazole as active ingredient and b) at least 5% by weight (wt%) pregelatinized starch (PGS), wherein said composition is prepared by a method comprising dry granulation of a blend of the components to produce granules, and compression of the granules to a tablet.

2. The pharmaceutical composition according to claim 1, wherein said composition is free of hydroxypropyl cellulose.

3. The pharmaceutical composition according to claim 1, wherein the active ingredient has a particle size distribution (D90) of less than or equal to 20 µm.

4. The pharmaceutical composition according to any one of the preceding claims, wherein the composition further comprises a water-soluble filler, a water-insoluble filler and a lubricant.

5. The pharmaceutical composition according to claim 4, wherein the water-soluble filler is a sugar, preferably lactose.

6. The pharmaceutical composition according to claim 4, wherein the water-insoluble filler is microcrystalline cellulose.

7. The pharmaceutical composition according to claim 4, wherein the lubricant is magnesium stearate.

8. The pharmaceutical composition according to any one of the preceding claims, comprising:
a. brexpiprazole, present in an amount of 0.01-25 wt%, preferably 1-10 wt%,
b. pregelatinized starch, present in an amount of 5-70 wt%, preferably 15-60 wt%.
c. water-soluble filler, preferably a sugar, more preferably lactose, present in an amount of 10-99 wt%, preferably 15-50 wt%,
d. water-insoluble filler, preferably microcrystalline cellulose, present in an amount of 5-70 wt%, preferably 5-50 wt%
e. lubricant, preferably magnesium stearate, present in an amount of 0.01-5 wt%, preferably 0.1-2 wt%, based on the total weight of all components of the tablet.

9. The pharmaceutical composition according to any one of the preceding claims, wherein the tablet is coated, preferably wherein the coating is a film coating comprising hydroxypropyl methyl cellulose (HPMC, hypromellose), preferably present in an amount of 0.1-5 wt% based on the total weight of all components of the tablet.

10. The pharmaceutical composition according to any one of the preceding claims, wherein the tablet shows at least 75% dissolution of the active ingredient after 30 minutes using method 2 of dissolution test of the US Pharmacopeia (USP), with a paddle speed of 50 revolutions per minute in 900mL of 0.05 M Acetate buffer at pH 4.3.

11. The pharmaceutical composition according to any one of the preceding claims for use as a medicament in the treatment of schizophrenia, major depression, and other central nervous system disorders.

12. Method of preparing a pharmaceutical composition in the form of a tablet, comprising dry granulation of a blend of the components of at least claim 1, preferably additionally according to one or more of claims 4-6, to produce granules and compression of the granules to a tablet.

13. Method of preparing a pharmaceutical composition in the form of a tablet according to the preceding claim, wherein the method comprises:
a. blending of the components of at least claim 1, preferably additionally according to one or more of claims 4-6, to produce a blend;
b. optionally compacting said blend into flakes;
c. milling the blend (or optionally the flakes) to produce granules;
d. lubricating the granules; and
e. compression of the granules to a tablet.

14. Method of preparing a pharmaceutical composition in the form of a tablet according to the preceding claim, wherein the method comprises additionally coating said tablet with a film coating, preferably comprising hydroxypropyl methyl cellulose (HPMC, hypromellose).

## Patentansprüche

1. Pharmazeutische Zusammensetzung in Form einer Tablette, umfassend a) Brexpiprazol als Wirkstoff und b) mindestens 5 Gew .-% vorgelatinierte Stärke (PGS), wobei die Zusammensetzung durch ein Verfahren umfassend eine Trockengranulierung einer Mischung der Komponenten zur Herstellung von einem Granulat und eine Verpressung des Granulats zu einer Tablette hergestellt wird.

2. Pharmazeutische Zusammensetzung nach Anspruch 1, wobei die Zusammensetzung frei von Hydroxypropylcellulose ist.

3. Pharmazeutische Zusammensetzung nach Anspruch 1, wobei der Wirkstoff eine Teilchengrößenverteilung (D90) von weniger als oder gleich 20 µm aufweist.

4. Pharmazeutische Zusammensetzung nach einem der vorhergehenden Ansprüche, wobei die Zusammensetzung ferner einen wasserlöslichen Füllstoff, einen wasserunlöslichen Füllstoff und ein Schmiermittel umfasst.

5. Pharmazeutische Zusammensetzung nach Anspruch 4, wobei der wasserlösliche Füllstoff ein Zucker ist, vorzugsweise Lactose.

6. Pharmazeutische Zusammensetzung nach Anspruch 4, wobei der wasserunlösliche Füllstoff mikrokristalline Cellulose ist.

7. Pharmazeutische Zusammensetzung nach Anspruch 4, wobei das Schmiermittel Magnesiumstearat ist.

8. Pharmazeutische Zusammensetzung nach einem der vorhergehenden Ansprüche, umfassend:
a. Brexpiprazol, vorhanden in einer Menge von 0,01 bis 25 Gew .-%, vorzugsweise 1 bis 10 Gew .-%,
b. vorgelatinierte Stärke, vorhanden in einer Menge von 5 bis 70 Gew .-%, vorzugsweise 15 bis 60 Gew .-%,
c. einen wasserlöslichen Füllstoff, bevorzugt einen Zucker, besonders bevorzugt Lactose, vorhanden in einer Menge von 10 bis 99 Gew .-%, bevorzugt 15 bis 50 Gew .-%,
d. einen wasserunlöslichen Füllstoff, vorzugsweise mikrokristalline Cellulose, vorhanden in einer Menge von 5 bis 70 Gew .-%, vorzugsweise 5 bis 50 Gew .-%,
e. ein Schmiermittel, vorzugsweise Magnesiumstearat, vorhanden in einer Menge von 0,01 bis 5 Gew .-%, vorzugsweise 0,1 bis 2 Gew .-%,
jeweils bezogen auf das Gesamtgewicht aller Komponenten derTablette.

9. Pharmazeutische Zusammensetzung nach einem der vorhergehenden Ansprüche, wobei die Tablette beschichtet ist, wobei die Beschichtung vorzugsweise eine Filmbeschichtung ist, welche Hydroxypropylmethylcellulose (HPMC, Hypromellose) umfasst, die vorzugsweise in einer Menge von 0,1 bis 5 Gew .-% vorliegt, bezogen auf das Gesamtgewicht aller Komponenten der Tablette.

10. Pharmazeutische Zusammensetzung nach einem der vorhergehenden Ansprüche, wobei die Tablette eine mindestens 75%ige Wirkstofffreisetzung nach 30 Minuten unter Verwendung der Methode 2 für den Dissolutionstest nach dem US-Amerikanischen Arzneibuch (US Pharmacopeia, USP) mit einer Blattrührer-Drehzahl von 50 Umdrehungen pro Minute in 900 ml 0,05 M Acetatpuffer bei pH 4,3 zeigt.

11. Pharmazeutische Zusammensetzung nach einem der vorhergehenden Ansprüche zur Verwendung als Medikament bei der Behandlung von Schizophrenie, Major-Depression und anderen Störungen des Zentralnervensystems.

12. Verfahren zur Herstellung einer pharmazeutischen Zusammensetzung in Form einer Tablette, umfassend die Trockengranulierung einer Mischung der Komponenten nach mindestens Anspruch 1, vorzugsweise zusätzlich nach einem oder mehreren der Ansprüche 4 bis 6, zur Herstellung eines Granulats und Verpressung des Granulats zu einer Tablette.

13. Verfahren zur Herstellung einer pharmazeutischen Zusammensetzung in Form einer Tablette nach dem vorhergehenden Anspruch, wobei das Verfahren umfasst:
a. Mischen der Komponenten nach mindestens Anspruch 1, vorzugsweise zusätzlich nach einem oder mehreren der Ansprüche 4 bis 6, zur Herstellung einer Mischung;
b. gegebenenfalls Kompaktieren der Mischung zu Schülpen;
c. Mahlen der Mischung (oder gegebenenfalls der Schülpen) zur Herstellung vom Granulat;
d. Schmieren des Granulats; und
e. Verpressen des Granulats zu einer Tablette.

14. Verfahren zur Herstellung einer pharmazeutischen Zusammensetzung in Form einer Tablette nach dem vorhergehenden Anspruch, wobei das Verfahren das zusätzliche Beschichten der Tablette mit einer Filmbeschichtung umfasst, die vorzugsweise Hydroxypropylmethylcellulose (HPMC, Hypromellose) umfasst.

## Revendications

1. Composition pharmaceutique sous forme de comprimé comprenant a) du brexpiprazole en tant que principe actif et b) au moins 5% en poids d'amidon prégélatinisé (PGS), dans laquelle ladite composition est préparée par un procédé comprenant la granulation à sec d'un mélange des composants pour produire des granulés et compression des granulés dans un comprimé.

2. Composition pharmaceutique selon la revendication 1, dans laquelle ladite composition est exempte d'hydroxypropylcellulose.

3. Composition pharmaceutique selon la revendication 1, dans laquelle l'ingrédient actif a une distribution de taille des particules (D90) inférieure ou égale à 20 µm.

4. Composition pharmaceutique selon l'une quelconque des revendications précédentes, dans laquelle la composition comprend en outre un agent diluant soluble dans l'eau, un agent diluant insoluble dans l'eau et un agent lubrifiant.

5. Composition pharmaceutique selon la revendication 4, dans laquelle l'agent diluant soluble dans l'eau est un sucre, de préférence du lactose.

6. Composition pharmaceutique selon la revendication 4, dans laquelle l'agent diluant insoluble dans l'eau est la cellulose microcristalline.

7. Composition pharmaceutique selon la revendication 4, dans laquelle l'agent lubrifiant est du stearate de magnésium.

8. Composition pharmaceutique selon l'une quelconque des revendications précédentes, comprenant :
a. le brexpiprazole, présent en une quantité de 0,01 à 25% en poids, de préférence de 1 à 10% en poids,
b. amidon prégélatinisé, présent en une quantité de 5 à 70% en poids, de préférence de 15 à 60% en poids,
c. l'agent diluant soluble dans l'eau, de préférence un sucre, plus préférablement du lactose, présente en une quantité de 10 à 99% en poids, de préférence de 15 à 50% en poids,
d. l'agent diluant insoluble dans l'eau, de préférence de la cellulose microcristalline, présente en une quantité de 5 à 70% en poids, de préférence de 5 à 50% en poids,
e. l'agent lubrifiant, de préférence du stéarate de magnésium, présent en une quantité de 0,01 à 5% en poids, de préférence de 0,1 à 2% en poids,
sur la base du poids total de tous les composants du comprimé.

9. Composition pharmaceutique selon l'une quelconque des revendications précédentes, dans laquelle le comprimé est enrobé, de préférence dans lequel l'enrobage est un pelliculage comprenant de l'hydroxypropylméthylcellulose (HPMC, hypromellose), présente de préférence dans une quantité de 0,1 à 5% en poids total de tous les composants du comprimé.

10. Composition pharmaceutique selon l'une quelconque des revendications précédentes, dans laquelle le comprimé présente une dissolution d'au moins 75% de l'ingrédient actif au bout de 30 minutes en utilisant le procédé 2 du test de dissolution de la pharmacopée américaine (USP), avec une vitesse de rotation des pâles de 50 tours par minute dans 900 ml de tampon acétate 0,05 M à pH 4,3.

11. Composition pharmaceutique selon l'une quelconque des revendications précédentes, destinée à être utilisée comme médicament dans le traitement de la schizophrénie, de la dépression majeure et d'autres troubles du système nerveux central.

12. Procédé de préparation d'une composition pharmaceutique sous forme de comprimé, comprenant la granulation à sec d'un mélange des composants d'au moins la revendication 1, de préférence en outre selon l'une ou plusieurs des revendications 4 à 6, pour produire des granulés et compression des granulés dans un comprimé.

13. Procédé de préparation d'une composition pharmaceutique sous la forme d'un comprimé selon la revendication précédente, dans lequel le procédé comprend :
a. mélanger des composants d'au moins la revendication 1, de préférence en outre selon une ou plusieurs des revendications 4 à 6, pour produire un mélange ;
b. éventuellement compacter ledit mélange en croûtes ;
c. broyer le mélange (ou éventuellement les croûtes) pour produire des granulés ;
d. lubrifier les granulés ; et
e. comprimer des granulés dans un comprimé.

14. Procédé de préparation d'une composition pharmaceutique sous la forme d'un comprimé selon la revendication précédente, dans lequel le procédé comprend en outre l'enrobage dudit comprimé avec un pelliculage, comprenant de préférence de l'hydroxypropylméthylcellulose (HPMC, hypromellose).
